# EUROPEAN PATENT APPLICATION

(11) **EP 2 062 579 A1**
(43) Date of publication of application: **27.05.2009**
(21) Application number: 07807115.6
(22) Date of filing: 12.09.2007
(51) Int. Cl.: A61K 31/4184, A61K 9/16, A61K 9/20, A61K 47/02, A61K 47/20, A61K 47/26, A61P 1/00, A61P 1/08

(54) **SOLID PHARMACEUTICAL COMPOSITION FOR ORAL ADMINISTRATION COMPRISING OPTICALLY STABLE RAMOSETRON**

(30) Priority: 15.09.2006 JP 2006250341
(71) Applicant: Astellas Pharma Inc., Tokyo 103-8411 (JP)
(72) Inventor: UMEJIMA, Hiroyuki, Tokyo 103-8411 (JP); KURIMOTO, Ippei, Tokyo 103-8411 (JP); KANBAYASHI, Atsushi, Tokyo 103-8411 (JP); MORI, Chieko, Tokyo 103-8411 (JP)
(74) Representative: HOFFMANN EITLE
(86) International application number: PCT/JP2007/067706
(87) International publication number: WO 2008/032726

(57) **Abstract**

Provided is a preparation of ramosetron which is stable under irradiation with light. The solid pharmaceutical composition of the present invention can provide a stable preparation by blending a compound absorbing light having a specific wavelength with ramosetron which is unstable, usually under irradiation with light, or a pharmaceutically acceptable salt thereof. Particularly, this technique is useful because it is adaptable to a preparation containing ramosetron or a pharmaceutically acceptable salt thereof at a low content or an orally disintegrating tablet containing ramosetron or a pharmaceutically acceptable salt thereof. Also, the present invention relates to a method for stabilizing a solid pharmaceutical composition of ramosetron or a pharmaceutically acceptable salt thereof, which is **characterized by** blending a compound having characteristics of absorbing light having a specific wavelength.

## Description

### Technical Field

The present invention relates to a stable solid pharmaceutical composition of ramosetron or a pharmaceutically acceptable salt thereof, which is characterized by containing a compound having characteristics of absorbing light at a specific wavelength. Also, the present invention relates to a method for stabilizing a solid pharmaceutical composition of ramosetron or a pharmaceutically acceptable salt thereof, which is characterized by blending a compound having characteristics of absorbing light having a specific wavelength.

### Background Art

A chemical name of ramosetron is (-)-(R)-5-[(1-methyl-1H-indol-3-yl)carbonyl]-4,5,6,7-tetrahydro-1H-benzimidazole. A series of tetrahydrobenzimidazole derivatives including said ramosetron and pharmaceutically acceptable salts thereof are reported as a pharmaceutical compound having an excellent antagonistic action for a serotonin (5-HT₃) receptor and suppressing digestive tract symptoms induced by administration of an anticancer agent, such as nausea and vomiting (see Patent Document 1), and in particular, a hydrochloride of ramosetron is already commercially available (hereinafter, the commercially available pharmaceutical compound will be referred to as "ramosetron hydrochloride"). It is known that the ramosetron hydrochloride exhibits an excellent pharmacological effect on adults upon its oral administration of 0.1 mg once a day, and it is commercially available under a trade name of "Nasea OD Tablets 0.1 mg" from Astellas Pharma Inc.
Also, a serotonin receptor antagonist is expected to be applicable as a therapeutic agent for irritable bowel syndrome (IBS). When an applicable disease is irritable bowel syndrome, the dose of ramosetron or a pharmaceutically acceptable salt thereof is considered to be effective in a range of from 0.001 to 0.05 mg in terms of the daily dose from the results of a clinical test, although it may differ depending on the ages or races of a patient (see Patent Document 2).
In general, in the case of formulating a pharmaceutical compound, the lower the content becomes, the more likely the pharmaceutical compound suffers from an interaction with pharmaceutical additives, and therefore, the pharmaceutical compound may have a problem of decrease in its stability.
As a technology for stabilizing ramosetron from temperature/humidity conditions, a composition obtained by blending a specific compound having a carbonyl group is known (see Patent Document 2).
On the other hand, as a technology for stabilizing ramosetron against light, a composition obtained by blending yellow ferric oxide, red ferric oxide, and titanium oxide is known (see Patent Document 2). However, this method required that red ferric oxide or yellow ferric oxide be contained in an amount of about 1% by weight in the formulation in order to achieve a sufficient stabilizing effect. These bases are hardly soluble in water, and accordingly, a method for dispersing them by physical mixing in the preparation must be performed. It is expected that the light stabilizing effect will be increased by further increasing the addition amount, but there is possibility that sticking occur on tableting or that an interaction between a drug and a base occurs. Therefore, it is considered preferable that the amount of these additives is as low as possible.
Also, in addition to blend yellow ferric oxide, since a 0.1 mg tablet of ramosetron hydrochloride as a product employs a packaging form of a colored polypropylene film and a PTP sheet of an aluminum flake, it has no problem as a commercial product before opening the package, but there is a need of packing it in a pack with a drug to be combined by means of an automatic packing machine, and there is also a need of consideration of light stabilization after opening the packaging. Generally, in order to assure the stability after opening the packaging of a light-unstable drug, a method is usually considered, in which a component mixture obtained by dispersing and blending a coloring agent is used to make a physically light-proof preparation form such as tablet, film-coated, sugar-coated, or capsule preparation, and the like, thereby improving the stability. (see Non-Patent Document 1).
However, in the case of an orally disintegrating tablet, since the hardness of a tablet is lower than that of a conventional tablet, it is difficult to perform film coating used for conventional tablets, and there is a demand for a new light stabilization technology.
Thus, there remains a need to improve a stabilization technology after opening the packaging of ramosetron, in particular, the stabilization technology against light, and particularly, it has been thought that the stabilization technology is insufficient for a low content preparation and/or an orally disintegrating tablet.
On the other hand, when a light-unstable pharmaceutical substance is stabilized against light, there is known a method in which a substance having a similar light absorbance behavior to that of the pharmaceutical substance to be protected is added (see Patent Document 3).
However, there exists an example that even in a case where an additive having a similar light-absorbing maximum wavelength to that of a pharmaceutical substance to be protected is used, the pharmaceutical substance cannot be stabilized against light, and stabilization by means of an additive having a similar light-absorbing behavior is not common. Specifically, it is known that troxerutin as a light stabilizer has a small stabilizing effect on nifedipine that is a light-unstable pharmaceutical substance showing a similar light-absorbing behavior, but it has a stabilizing effect on molsidomine having little similarity in the light-absorbing behavior (see Patent Document 4). In the above-described Non-Patent Document 1, there is a description on a sulfisomidine tablet. Although it shows maximum absorbances at 266 nm and 347 nm, the decomposition rate constant is increased at a lower wavelength with respect to the decomposition (in the reference, the shortest wavelength is 250 nm), and thus there is indicated that there is no relationship between the absorbance wavelength and the decomposition.
Furthermore, there is described a method in which when a light-unstable drug is stabilized, Food Yellow Nos. 4 and 5, Food Red Nos. 3 and 102, iron sesquioxide, and titanium oxide, or the like are added as a coloring agent in the preparation process, but in fact, it relates to a sofalcone-containing preparation that contains Food Yellow No. 5 or iron sesquioxide, and it cannot be said that the method has a stabilizing effect with respect to any of drugs. (See Patent Document 5)
In addition, there is described a method in which when a light-unstable nifedipine is stabilized, Food Yellow No. 5 is uniformly dispersed in the outer film of a soft capsule. (See Patent Document 6)
As such, the stabilizing method varies depending on the compounds to be stabilized, and the stabilizing method of the present invention in ramosetron having a structure different from the structure for the above-described prior art cannot be expected by a person skilled in the art.
Patent Document 1: Specification of European Patent No. 381422
Patent Document 2: Pamphlet of International Publication No. 04/066998
Patent Document 3: JP-A-58-57322
Patent Document 4: JP-A-60-156678
Patent Document 5: JP-A-2000-191516
Patent Document 6: JP-A-55-22645
Non-Patent Document 1: MATSUDA YOSHIHISA, et al., "Recent preparation technologies and their applications I", published by Medical Journal, September 1, 1983, p. 121 to 123

### DISCLOSURE OF THE INVENTION

### PROBLEM THAT THE INVENTION IS TO SOLVE

Thus, a sufficient stabilizing effect against light cannot be obtained merely by blending titanium oxide or iron sesquioxide, and there is particularly a desire for a preparation containing ramosetron at a low content and/or an orally disintegrating tablet containing ramosetron, under irradiation with light.

### MEANS FOR SOLVING THE PROBLEM

After studies on a preparation which is optimum to indications for which an effect is expected at a low dose, such as irritable bowel syndrome, and it was found that when stored under irradiation with light, and under a high-temperature and high-humidity condition, ramosetron or a pharmaceutically acceptable salt thereof is lowered with respect to its assay value, and is liable to be decomposed.
Then, for the sake of developing a preparation of ramosetron or a pharmaceutically acceptable salt thereof that is stable even at a low content, the present inventors have made extensive investigations, and as a result, they have found that mannitol and Red No. 3 did not exhibit a remarkable stabilizing effect against light, but αG hesperidin, methyl hesperidin, Red No. 102, and sodium azulene sulfonate exhibited a remarkable stabilizing effect against light.
Next, the present inventors have analyzed the light absorbance characteristics of a group of the compounds exhibiting a stabilizing effect against light and a group of the compounds not exhibiting a stabilizing effect against light. The light absorbance spectrum of ramosetron has three peaks showing maximum absorbance wavelengths at 210 nm, 249 nm, and 311 nm (see Fig. 1 as described later). When they have analyzed the test results of the light stability of ramosetron and the absorbance spectrum of each of the compounds, they found that ramosetron is stabilized depending on the area under the spectrum curve around the valley portion between the maximum peaks as shown at the wavelength range of 220 nm to 240 nm, and/or at the wavelength range of 280 nm to 300 nm (see Figs. 3 and 4 as described later).
In addition, surprisingly, the present inventors have also found that when a substance selected from a group of the flavonoid compounds consisting of αG hesperidin and methyl hesperidin is added to ramosetron or a pharmaceutically acceptable salt thereof, there gives a remarkable stabilizing effect under a high-temperature and high-humidity condition, thereby completing the present invention.

Specifically, the present invention provides the followings:
1. a solid pharmaceutical composition of ramosetron or a pharmaceutically acceptable salt thereof, which contains one, or two or more kinds of the compounds selected from the group consisting of the compounds having 4.5 or more of the area under the spectrum curve at the wavelength range of 220 nm to 240 nm and/or 2.5 or more of the area under the spectrum curve at the wavelength range of 280 nm to 300 nm in a 0.001 w/v% aqueous solution thereof,
2. the pharmaceutical composition as described in Claim 1, wherein the compounds having 4.5 or more of the area under the spectrum curve at the wavelength range of 220 nm to 240 nm, and/or 2.5 or more of the area under the spectrum curve at the wavelength range of 280 nm to 300 nm in a 0.001 w/v% aqueous solution thereof is αG hesperidin, methyl hesperidin, Food Red No. 102, or sodium azulene sulfonate,
3. the pharmaceutical composition as described in Claim 1 or 2, wherein the blending amount of one, or two or more kinds of the compounds selected from the group consisting of the compounds having 4.5 or more of the area under the spectrum curve at the wavelength range of 220 nm to 240 nm and/or 2.5 or more of the area under the spectrum curve at the wavelength range of 280 nm to 300 nm in a 0.001 w/v% aqueous solution thereof is from 0.001 to 90% by weight in the formulation,
4. the pharmaceutical composition as described in Claim 3, wherein the blending amount of ramosetron or a pharmaceutically acceptable salt thereof is from 0.0001 to 0.5% by weight in the formulation,
5. a particulate pharmaceutical composition, wherein ramosetron or a pharmaceutically acceptable salt thereof is coated with one, or two or more kinds of the compounds selected from the group consisting of the compounds having 4.5 or more of the area under the spectrum curve at the wavelength range of 220 nm to 240 nm and/or 2.5 or more of the area under the spectrum curve at the wavelength range of 280 nm to 300 nm,
6. an orally disintegrating tablet, which contains the pharmaceutical composition as described in any one of Claims 1 to 5,
7. the pharmaceutical composition as described in any one of Claims 1 to 6, which further contains one, or two or more selected from the group consisting of yellow ferric oxide, red ferric oxide, and titanium oxide in an amount of 0.0001 to 0.5% by weight in the formulation,
8. a method for stabilizing a solid pharmaceutical composition of ramosetron or a pharmaceutically acceptable salt thereof, which comprises blending one or two or more kinds of the compounds selected from the group consisting of the compounds having 4.5 or more of the area under the spectrum curve at the wavelength range of 220 nm to 240 nm and/or 2.5 or more of the area under the spectrum curve at the wavelength range of 280 nm to 300 nm in a 0.001 w/v% aqueous solution thereof.

Thus, for stabilization of ramosetron or a pharmaceutically acceptable salt thereof against light, there has been remarkable difference in the stabilizing effects according to the kinds of the food colorant to be added. As described in Patent Document 5, it is impossible to simply add a food colorant, it is necessary to precisely examine the light absorbing property of the food colorant, and thus, the effect cannot be predicted from the prior art.
Further, for stabilization of ramosetron or a pharmaceutically acceptable salt thereof against light, it was impossible that the effect cannot be achieved with Food Red No. 3 (the absorbance maximum wavelength was far from that of the ramosetron hydrochloride only by 12 nm) that shows a similar absorbance behavior to that of the drug. Accordingly, as described in Patent Document 3, it can be said that stabilization is difficult with an additive having a similar light absorbance behavior, and an additive effective for stabilization cannot be predicted.

### EFFECTS OF THE INVENTION

The solid pharmaceutical composition of the present invention can provide a stable preparation by blending a compound absorbing a light having a specific wavelength with ramosetron which is unstable, mainly under irradiation with light, or a pharmaceutically acceptable salt thereof. Particularly, it is useful as a technique that is adaptable to a preparation containing ramosetron or a pharmaceutically acceptable salt thereof at a low content or an orally disintegrating tablet containing ramosetron or a pharmaceutically acceptable salt thereof.

### BRIEF DESCRIPTION OF THE DRAWINGS

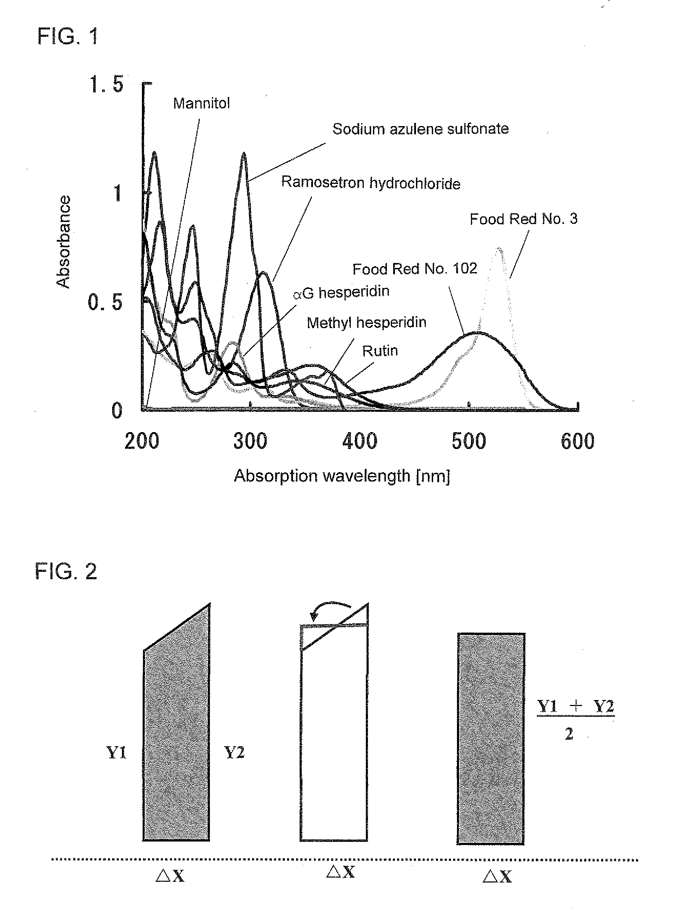

[Fig. 1] Fig. 1 is a graph showing the relationship between the absorbance wavelength and the absorbance of various compounds. The measurement method is as follows. A 0.001 w/v% aqueous solution of a drug and various stabilizers was prepared, and the absorbances at each absorbance wavelength in a range of 200 nm to 600 nm were measured. For measurement, a UV meter (MPS-2450; manufactured by: SHIMADZU Corporation) was used. Condition: scan speed: medium speed, sampling pitch: 1.0, length of light path: 10 mm, width of a slit: 2.0 nm.
[Fig. 2] Fig. 2 is a graph showing a method for calculating the area under the spectrum curve of various compounds. The area under the spectrum curve was calculated by a trapezoidal formula. That is, for the spectrum of each of the compounds, the trapezoidal area was calculated at every sampling pitch (1.0 nm), and a desired area was measured from the total value of the trapezoidal areas in the wavelength range.

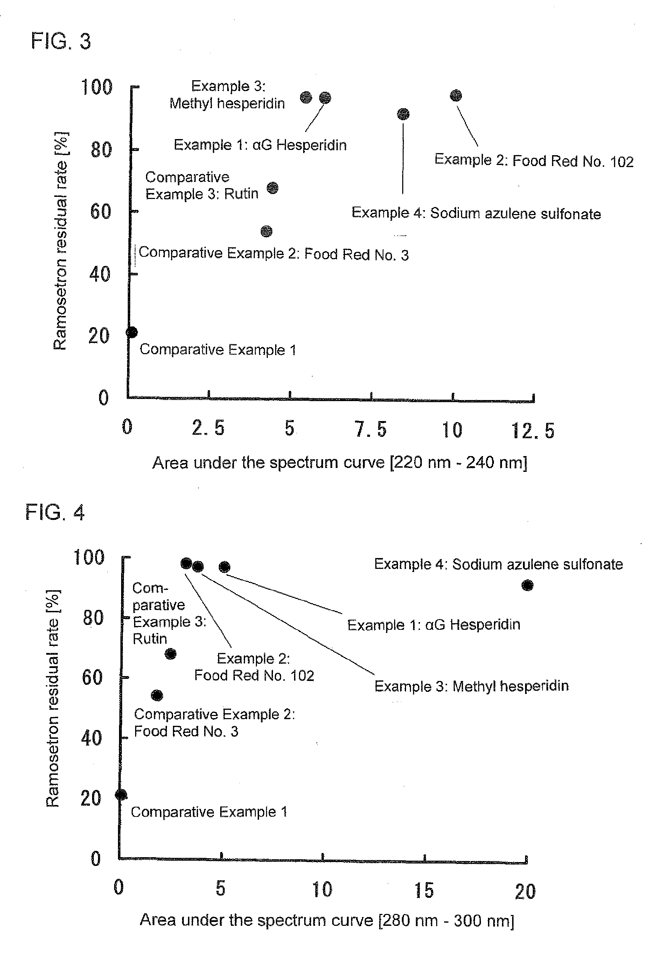

[Fig. 3] Fig. 3 is a graph showing the relationship between the area under the spectrum curve at the wavelength range of 220 nm to 240 nm of various compounds and the residual rate of ramosetron.
[Fig. 4] Fig. 4 is a graph showing the relationship between the area under the spectrum curve at the wavelength range of 280 nm to 300 nm of various compounds and the residual rate of ramosetron.

### BEST MODE FOR CARRYING OUT THE INVENTION

A pharmaceutical composition of the present invention will be described below.
Ramosetron to be used in the present invention is a pharmaceutical compound having the foregoing chemical name and described in Example 44 of JP-B-6-25153, and the like, and specific examples of a pharmaceutically acceptable salt thereof include salts of mineral acids such as hydrochloric acid, sulfuric acid, phosphoric acid, hydrobromic acid, and the like; salts of organic acids such as acetic acid, oxalic acid, succinic acid, citric acid, maleic acid, malic acid, fumaric acid, tartaric acid, methanesulfonic acid, and the like; and salts of acidic amino acid such as glutamic acid, aspartic acid, and the like. Of these, commercially available ramosetron hydrochloride is preferable. Also, ramosetron or a pharmaceutically acceptable salt thereof can be easily obtained according to the preparation method described in the above-cited patent document.
The amount of ramosetron or a pharmaceutically acceptable salt thereof to be used is not particularly limited so far as it is an effective amount. In particular, though it was found that ramosetron or a pharmaceutically acceptable salt thereof is unstable against temperature/humidity in a low content preparation, it is estimated that this matter is a substantially inherent problem even in a high content preparation, and therefore, a similar stabilizing effect can be expected. Accordingly, the use amount thereof is not limited to an effective amount against adaptation diseases of irritable bowel syndrome, but it includes effective amounts of the conventional products that are commercially available. Concretely, the blending amount of ramosetron or a pharmaceutically acceptable salt thereof is preferably from 0.0001 to 0.5% by weight, more preferably from 0.0001 to 0.25% by weight, and further preferably from 0.0005 to 0.05% by weight in the preparation. Also, when the amount of ramosetron or a pharmaceutically acceptable salt thereof to be used is expressed in terms of a unit preparation, it is specifically from 0.1 to 500 µg, more preferably from 0.1 to 250 µg, and further preferably from 1 to 50 µg.

The compound for stabilizing ramosetron or a pharmaceutically acceptable salt thereof against light used in the present invention is a compound having 4.5 or more of the area under the spectrum curve at the wavelength range of 220 nm to 240 nm or more in a 0.001 w/v% aqueous solution thereof, and/or a compound having 2.5 or more of the area under the spectrum curve at the wavelength range of 280 nm to 300 nm in a 0.001 w/v% aqueous solution thereof, as described above. It is not particularly limited so far as it stabilizes ramosetron or a pharmaceutically acceptable salt thereof under irradiation with light. It is more preferably a compound having 5.0 or more of the area under the spectrum curve at the wavelength range of 220 nm to 240 nm in a 0.001 w/v% aqueous solution thereof, and/or a compound having 2.7 or more of the area under the spectrum curve at the wavelength range of 280 nm to 300 nm in a 0.001 w/v% aqueous solution thereof. It is most preferably a compound having 5.5 or more of the area under the spectrum curve at the wavelength range of 220 nm to 240 nm in a 0.001 w/v% aqueous solution thereof, and/or 3.1 or more of the area under the spectrum curve at the wavelength range of 280 nm to 300 nm in a 0.001 w/v% aqueous solution thereof. Specific examples of the compound used in the present invention for stabilizing ramosetron against light include αG hesperidin, methyl hesperidin, Food Red No. 102, sodium azulene sulfonate, tannic acid, sodium copper chlorophyllin, Food Yellow No. 4, Food Red No. 106, Food Red No. 40, and Food Red No. 2, preferably αG hesperidin, methyl hesperidin, Food Red No. 102, and sodium azulene sulfonate, more preferably αG hesperidin, methyl hesperidin, and Food Red No. 102, and particularly preferably αG hesperidin and methyl hesperidin. These compounds can be suitably used singly or in combination of two or more kinds thereof.
Further, the compound for stabilizing ramosetron or a pharmaceutically acceptable salt thereof against light used in the present invention can further contain/blend one, or two or more kinds selected from the group consisting of yellow ferric oxide, red ferric oxide, and titanium oxide, within a range which causes neither sticking on tableting, nor an interaction between a drug and a base, and specifically, contain/blend in an amount of 0.0001 to 0.5% by weight in the formulation.

On the other hand, the compound for stabilizing ramosetron or a pharmaceutically acceptable salt thereof against a high-temperature and high-humidity condition used in the present invention is in a group of flavonoid compounds for stabilizing ramosetron or a pharmaceutically acceptable salt thereof. Specific examples of the group of flavonoid compounds include apigenin, quercetin, apiin, hesperidin, citronin, daizin, rutin, and naringin, preferably hesperidin and rutin, and more preferably hesperidin. These compounds can be suitably used singly or in combination of two or more kinds thereof.
The blending amount of the compound for stabilizing ramosetron or a pharmaceutically acceptable salt thereof against light, or a high-temperature and high-humidity condition is not limited so far as it allows stabilization, but it is specifically from 0.001 to 90% by weight, preferably from 0.01 to 50% by weight, and more preferably from 0.05 to 20% by weight.
Further, with respect to ramosetron of the present invention, a particulate pharmaceutical composition, which is characterized by being coated with one, or two or more kinds selected from the group consisting of a compound having 4.5 or more of the area under the spectrum curve at the wavelength range of 220 nm to 240 nm or more in a 0.001 w/v% aqueous solution thereof, and/or a compound having 2.5 or more of the area under the spectrum curve at the wavelength range of 280 nm to 300 nm in a 0.001 w/v% aqueous solution thereof is a drug-coated particle as described below, and it can be used as powder and granules, as well as it can be contained in a tablet, a film-coated tablet, an orally disintegrating tablet, or the like. If the pharmaceutical composition of the present invention is a particle such as granulates, the particle diameter of the particulate pharmaceutical composition is not particularly limited so far as the maximum diameter is no more than 2 mm. If it is contained in an intraorally quick disintegrating tablet, the particle diameter is not particularly limited so far as it does not give any unpleasant feeling like a sandy taste upon taking the composition, but the composition is preferably prepared with an average particle diameter of 350 µm or less. The average particle diameter is more preferably from 1 to 350 µm, and particularly preferably from 20 to 350 µm. As for the particle size distribution, the distribution is not particularly limited so far as the particle is suitable for coating such as masking of a bitter taste, and the like, and 80% of the total weight is preferably distributed in from 1 to 350 µm, 80% of the total weight is more preferably distributed in from 50 to 300 µm, and 80% of the total weight is particularly preferably distributed in from 100 to 250 µm.

Various pharmaceutical additives are properly used in the solid pharmaceutical composition of the present invention to make a preparation. Such the pharmaceutical additives are not particularly limited so far as they are pharmaceutically acceptable additives. For these, excipients, binders, disintegrating agents, sour agents, effervescent agents, artificial sweeteners, flavors, lubricants, coloring agents, and the like are used. Examples of the excipients include lactose, crystalline cellulose, microcrystalline cellulose, D-sorbitol, D-mannitol, and the like. Examples of the binders include hydroxypropylmethyl cellulose, hydroxypropyl cellulose, povidone, polyvinyl alcohol, methyl cellulose, gum arabic, and the like. Examples of the disintegrating agents include cornstarch, potato starch, carmellose, carmellose calcium, carmellose sodium, crosscarmellose sodium, low-substitution degree hydroxypropyl cellulose, crosspovidone, and the like. Examples of the sour agents include citric acid, tartaric acid, malic acid, and the like. Examples of blowing agents include sodium bicarbonate. Examples of the artificial sweeteners include saccharin sodium, glycyrrhizin dipotassium, aspartame, stevia, thaumatin, and the like. Examples of the flavors include lemon, lemon lime, orange, menthol, and the like. Examples of the lubricants include magnesium stearate, calcium stearate, sucrose fatty acid esters, polyethylene glycol, talc, stearic acid, and the like. The pharmaceutical additives can be suitably used singly or in combination of two or more kinds thereof. Examples of the coloring agents include red ferric oxide, yellow ferric oxide, Food Yellow Nos. 4 and 5, Food Blue No 3, and the like, and they are not particularly limited so far as these coloring agents are also intended to color.

Further, a compound having a specific carbonyl group can be further added for stabilization, in addition to ramosetron or a pharmaceutically acceptable salt thereof, even under a temperature/humidity condition, within a range not interfering with the effect of the present invention (see Patent Document 2). Specifically, examples thereof include an aliphatic carboxylic acid selected from the group consisting of maleic acid, malonic acid, succinic acid, and fumaric acid, or an ester thereof, a hydroxycarboxylic acid selected from the group consisting of tartaric acid, malic acid, and citric acid, or an ester thereof, an acidic amino acid that is aspartic acid or glutamic acid, an enolic acid that is ascorbic acid or erythorbic acid, an aromatic carboxyl compound that is phthalic acid or propyl gallate, or an ester thereof, a carboxyl group-containing high-molecular substance that is carboxymethyl cellulose, alginic acid, or the like.

Hereinbelow, a specific method for preparing the pharmaceutical composition of the present invention will be described.
The solid pharmaceutical composition of the present invention can be prepared by a per se known method, and can be formed into, for example, powder, granules, a tablet, a film-coated tablet, an orally disintegrating tablet, or the like, and is usually used for oral administration. With respect to disintegrable tablets in oral cavity, a lot of technologies are recently developed, but there are no particular limitations, for example, an orally disintegrating tablet can be formed according to the specifications of U.S. Patent Nos. 5,466,464, 5,576,014, and 6,589,554, and the pamphlets of WO 03/009831 and WO 02/092057, and the like.

As the method of adding a compound for stabilizing against light and/or for a high-temperature and high-humidity condition of the present invention to the preparation, a method of adding the compound in a process for preparing powder, granules, a tablet, a film-coated tablet, or an orally disintegrating tablet can be mentioned.
For example, the stabilizing compound of the present invention can be added to the preparation, by mixing ramosetron or a pharmaceutically acceptable salt thereof and a part or the whole of the compound for stabilizing against light of the present invention with an excipient, and the like, and then performing high wet granulation thereof using a binding agent in a granulation process, by performing wet granulation using a binding liquid containing ramosetron or a pharmaceutically acceptable salt thereof and a part or the whole of the stabilizing compound of the present invention, by performing wet granulation of powder obtained by mixing a part or the whole of the compound for stabilizing against light of the present invention with an excipient using a binding agent containing ramosetron or a pharmaceutically acceptable salt thereof, by performing wet granulation of powder obtained by mixing ramosetron or a pharmaceutically acceptable salt thereof with an excipient using a binding liquid containing a part or the whole of the stabilizing compound of the present invention, or by performing wet granulation of ramosetron or a pharmaceutically acceptable salt thereof and an excipient, and then mixing the resultant with a part or the whole of the stabilizing compound of the present invention.
Alternatively, the stabilizing compound of the present invention can be added to the preparation by simply mixing ramosetron or a pharmaceutically acceptable salt thereof and a part or the whole of the stabilizing compound of the present invention with an excipient, and the like.
In addition, a tablet of a matrix type containing ramosetron or pharmaceutically acceptable salt thereof, and a part or the whole of the stabilizing compound of the present invention can be prepared by making the prepared granule into a tablet with an excipient, and the like.

Moreover, in order to prepare powder of the present invention, ramosetron or a pharmaceutically acceptable salt thereof can be used itself as a core, but usually a fine particle to be a core containing ramosetron or a pharmaceutically acceptable salt thereof is previously prepared. A known technology can be applied for the preparation of a fine particle to be a core, and for example, by mixing ramosetron or a pharmaceutically acceptable salt thereof and a suitable excipient (for example, microcrystalline cellulose, lactose, corn starch, and the like), making the resultant into granules using a binding agent (for example, hydroxypropylcellulose, hydroxypropyl methylcellulose, sugars, and the like), sieving, and drying, or by spraying a liquid obtained by dissolving or dispersing ramosetron or a pharmaceutically acceptable salt thereof in a binding agent solution onto a particle to be a suitable core (for example, microcrystalline cellulose particle, white sugar granule, and the like), a particle can be prepared. Further, the particulate pharmaceutical composition of the present invention, having ramosetron or a pharmaceutically acceptable salt thereof coated with the stabilizing compound of the present invention can be prepared by spraying a coating liquid obtained by dissolving the stabilizing compound of the present invention in a solvent such as water, and the like, onto the prepared composition in a particle form containing ramosetron or a pharmaceutically acceptable salt thereof.
Further, by making the prepared particulate pharmaceutical composition of the present invention into a tablet with an excipient, and the like, a tablet containing granules having ramosetron or a pharmaceutically acceptable salt thereof coated with the stabilizing compound of the present invention can be prepared.

Further, the foregoing granulation can be carried out by means of known machines and methods, for example, fluidized bed granulation, high-speed shearing granulation, kneading granulation, extrusion granulation, or rotating granulation, or the like. Preferably, a granulated product suitable for tableting can be prepared by fluidizing the powders using a fluidized bed granulation method, and spraying a binding agent solution.
The tableting can be carried out by means of known machines and methods, for example, by a rotary tableting machine, a single-shot tableting machine, a high-speed centrifugal tableting machine, or the like.
The above coating process can be carried out by means of known machines and methods, for example, by a fluidized bed granulator, and the like, to fluidize the nuclear particles, and then to spray a solution containing ramosetron or a pharmaceutically acceptable salt thereof and/or the stabilizing compound of the present invention upward, downward, or sideward.
Upon granulation, in a case of performing wet granulation with a binding liquid containing ramosetron or a pharmaceutically acceptable salt thereof and a part or the whole of the stabilizing compound of the present invention, or of performing wet granulation of powder containing the stabilizing compound of the present invention with a binding liquid containing ramosetron or a pharmaceutically acceptable salt thereof, the amount is usually from 0.1 to 20% by weight, suitably from 0.2 to 10% by weight, and more suitably from 0.2 to 5% by weight, based on the total of the composition, taking the preparation thereof into consideration. For example, the process consists of a process of dissolving or suspending ramosetron or a pharmaceutically acceptable salt thereof, and if desired, an organic acid, and the stabilizing compound of the present invention in purified water, and a process for spraying the aqueous solution or suspension onto powder obtained by blending an excipient, and if desired, an organic acid, and a coloring agent in a wet granulator such as a fluidized bed granulator, and the like, and then drying. A pharmaceutically acceptable pharmaceutical additive may be uniformly dispersed to and added with the relevant aqueous solution or suspension and/or the powder to be fluidized. The relevant aqueous solution and the suspension are usually used at a concentration in terms of a binding agent that is used for performing wet granulation.

The method for stabilizing the solid pharmaceutical composition of ramosetron or a pharmaceutically acceptable salt thereof of the present invention can be carried out by the method as described in the description of the invention with regard to the above-described pharmaceutical composition.

### Examples

Hereinbelow, the present invention will be specifically described with reference to Examples, but the scope of the present invention is not limited thereto.

| Comparative Example 1 | |
|---|---|
| Ramosetron hydrochloride | 0.0036 part |
| Mannitol | 91.9 parts |
| Maltose | 6.9 parts |
| Citric acid anhydride | 0.2 part |
| Magnesium stearate | 1 part |

1.725 parts of maltose, 0.0036 part of ramosetron hydrochloride and 0.2 part of citric acid anhydride were dissolved in 6.9 parts of water using a magnetic stirrer under stirring to prepare a spraying liquid. Next, 91.9 parts of mannitol was put into a fluidized bed granulator (GPCG-1, manufactured by Powrex Corporation) equipped with a bug filter, and the spraying liquid was sprayed thereonto at an inlet temperature of 63°C, a spraying rate of 15 g/min, and a spraying/drying cycle of 12 seconds/24 seconds to conduct a fluid granulation. Further, a spraying liquid obtained by dissolving 5.175 parts of maltose in 20.7 parts of water was sprayed onto the granulated product under the same condition to conduct a fluid granulation. After granulation, the granulated product was dried for 1 minute, and 1 part of magnesium stearate was then mixed therewith. The mixed powder was made into a tablet using a rotary tableting machine at a rate of 70 mg per tablet. It was stored at 25°C and a relative humidity of 75% for 18 hours, and then stored at 30°C and a relative humidity of 40% for 4 hours to obtain a comparative orally disintegrating tablet of the preparation of the present invention.

### Comparative Example 2

| | |
|---|---|
| Ramosetron hydrochloride | 0.0035 part |
| Mannitol | 90.3 parts |
| Maltose | 6.8 parts |
| Citric acid anhydride | 0.2 parts |
| Food Red No. 3 | 1.8 parts |
| Magnesium stearate | 1 part |

To 97.3 parts of the granulated product as prepared in Comparative Example 1 were mixed 1.8 parts of Food Red No. 3 and 1 part of magnesium stearate in a polyethylene bag, and the mixture was made into a tablet using autograph (AGS-20kNG, manufactured by Shimadzu Co.) at a rate of 71.25 mg per tablet to obtain a comparative orally disintegrating tablet of the preparation of the present invention.

### Comparative Example 3

| | |
|---|---|
| Ramosetron hydrochloride | 0.0035 part |
| Mannitol | 91.2 part |
| Maltose | 6.8 parts |
| Citric acid anhydride | 0.2 part |
| Rutin | 0.9 part |
| Magnesium stearate | 1 part |

To 98.1 parts of the granulated product as prepared in Comparative Example 1 were mixed 0.9 part of rutin and 1 part of magnesium stearate in a polyethylene bag, and the mixture was made into a tablet using autograph (AGS-20kNG, manufactured by Shimadzu Co.) at a rate of 71.25 mg per tablet to obtain a comparative orally disintegrating tablet of the preparation of the present invention.

### Example 1

| | |
|---|---|
| Ramosetron hydrochloride | 0.002 part |
| Mannitol | 90.9 parts |
| Maltose | 6.9 parts |
| Citric acid anhydride | 0.2 part |
| αG Hesperidin | 1 part |
| Magnesium stearate | 1 part |

1.725 parts of maltose, 0.002 part of ramosetron hydrochloride and 0.2 part of citric acid anhydride were dissolved in 6.9 parts of water using a magnetic stirrer under stirring to prepare a spraying liquid. Next, 90.9 parts of mannitol was put into a fluidized bed granulator (GPCG-1, manufactured by Powrex Corporation) equipped with a bug filter, and the spraying liquid was sprayed thereonto at an inlet temperature of 63°C, a spraying rate of 15 g/min, and a spraying/drying cycle of 12 seconds/24 seconds to conduct a fluid granulation. Further, a spraying liquid obtained by dissolving 5.175 parts of maltose and 0.1 part of αG hesperidin in 20.7 parts of water was sprayed to the granulated product under the same condition to conduct a fluid granulation. After granulation, the granulated product was dried for 1 minute, and 1 part of magnesium stearate was then mixed therewith. The mixed powder was made into a tablet using a rotary tableting machine at a rate of 120 mg per tablet. It was stored at 25°C and a relative humidity of 75% for 18 hours, and then stored at 30°C and a relative humidity of 40% for 4 hours to obtain an orally disintegrating tablet of the preparation of the present invention.

### Example 2

| | |
|---|---|
| Ramosetron hydrochloride | 0.002 part |
| Mannitol | 91.4 parts |
| Maltose | 7 parts |
| Citric acid anhydride | 0.2 part |
| Food Red No. 102 | 0.5 part |
| Magnesium stearate | 1 part |

1.725 parts of maltose, 0.002 part of ramosetron hydrochloride and 0.2 part of citric acid anhydride were dissolved in 6.9 parts of water using a magnetic stirrer under stirring to prepare a spraying liquid. Next, 91.4 parts of mannitol was put into a fluidized bed granulator (GPCG-1, manufactured by Powrex Corporation) equipped with a bug filter, and the spraying liquid was sprayed thereonto at an inlet temperature of 63°C, a spraying rate of 15 g/min, and a spraying/drying cycle of 12 seconds/24 seconds to conduct a fluid granulation. Further, a spraying liquid obtained by dissolving 5.175 parts of maltose and 0.5 part of Food Red No. 102 in 20.7 parts of water was sprayed to the granulated product under the same condition to conduct a fluid granulation. After granulation, the granulated product was dried for 1 minute, and 1 part of magnesium stearate was then mixed therewith. The mixed powder was made into a tablet using a rotary tableting machine at a rate of 120 mg per tablet. It was stored at 25°C and a relative humidity of 75% for 18 hours, and then stored at 30°C and a relative humidity of 40% for 4 hours to obtain an orally disintegrating tablet of the preparation of the present invention.

### Example 3

| | |
|---|---|
| Ramosetron hydrochloride | 0.002 part |
| Mannitol | 91 parts |
| Maltose | 6.8 parts |
| Citric acid anhydride | 0.2 part |
| Methyl hesperidin | 1 part |
| Magnesium stearate | 1 part |

1.7 parts of maltose, 0.002 part of ramosetron hydrochloride and 0.2 part of citric acid anhydride were dissolved in 6.8 parts of water using a magnetic stirrer under stirring to prepare a spraying liquid. Next, 91 parts of mannitol was put into a fluidized bed granulator (GPCG-1, manufactured by Powrex Corporation) equipped with a bug filter, and the spraying liquid was sprayed thereonto at an inlet temperature of 63°C, a spraying rate of 15 g/min, and a spraying/drying cycle of 12 seconds/24 seconds to conduct a fluid granulation. Further, a spraying liquid obtained by dissolving 5.1 parts of maltose and 1 part of methyl hesperidin in 20.4 parts of water was sprayed to the granulated product under the same condition to conduct a fluid granulation. After granulation, the granulated product was dried for 1 minute, and 1 part of magnesium stearate was then mixed therewith. The mixed powder was made into a tablet using a rotary tableting machine at a rate of 120 mg per tablet. It was stored at 25°C and a relative humidity of 75% for 18 hours, and then stored at 30°C and a relative humidity of 40% for 4 hours to obtain an orally disintegrating tablet of the preparation of the present invention.

### Example 4

| | |
|---|---|
| Ramosetron hydrochloride | 0.002 part |
| Mannitol | 91 parts |
| Maltose | 6.8 parts |
| Citric acid anhydride | 0.2 part |
| Sodium azulene sulfonate | 1 part |
| Magnesium stearate | 1 part |

1.7 parts of maltose, 0.002 part of ramosetron hydrochloride and 0.2 part of citric acid anhydride were dissolved in 6.8 parts of water using a magnetic stirrer under stirring to prepare a spraying liquid. Next, 91 parts of mannitol was put into a fluidized bed granulator (GPCG-1, manufactured by Powrex Corporation) equipped with a bug filter, and the spraying liquid was sprayed thereonto at an inlet temperature of 63°C, a spraying rate of 15 g/min, and a spraying/drying cycle of 12 seconds/24 seconds to conduct a fluid granulation. Further, a spraying liquid obtained by dissolving 5.1 parts of maltose and 1 part of sodium azulene sulfonate in 20.4 parts of water was sprayed to the granulated product under the same condition to conduct a fluid granulation. After granulation, the granulated product was dried for 1 minute, and 1 part of magnesium stearate was then mixed therewith. The mixed powder was made into a tablet using a rotary tableting machine at a rate of 120 mg per tablet. It was stored at 25°C and a relative humidity of 75% for 18 hours, and then stored at 30°C and a relative humidity of 40% for 4 hours to obtain an orally disintegrating tablet of the preparation of the present invention.

### Example 5

The same process as in Example 1 was carried out, except that the amount of αG hesperidin was changed to 0.1 part, to obtain an orally disintegrating tablet of the preparation of the present invention.

### Example 6

The same process as in Example 1 was carried out, except that the amount of αG hesperidin was changed to 3 parts, to obtain an orally disintegrating tablet of the preparation of the present invention.

### Example 7

The same process as in Example 3 was carried out, except that the amount of methyl hesperidin was changed to 3 parts, to obtain an orally disintegrating tablet of the preparation of the present invention.

### Example 8

| | |
|---|---|
| Ramosetron hydrochloride | 0.002 part |
| Mannitol | 91 parts |
| Maltose | 6.8 parts |
| Citric acid anhydride | 0.2 part |
| Methyl hesperidin | 1 part |
| Yellow ferric oxide | 0.1 part |
| Magnesium stearate | 1 part |

1.7 parts of maltose, 0.002 part of ramosetron hydrochloride and 0.2 part of citric acid anhydride were dissolved in 6.8 parts of water using a magnetic stirrer under stirring to prepare a spraying liquid. Next, 91 parts of mannitol was put into a fluidized bed granulator (GPCG-1, manufactured by Powrex Corporation) equipped with a bug filter, and the spraying liquid was sprayed thereonto at an inlet temperature of 63°C, a spraying rate of 15 g/min, and a spraying/drying cycle of 12 seconds/24 seconds to conduct a fluid granulation. Further, a spraying liquid obtained by dissolving 5.1 parts of maltose and 1 part of methyl hesperidin in 20.4 parts of water was sprayed to the granulated product under the same condition to conduct a fluid granulation. After granulation, the granulated product was dried for 1 minute, and 0.1 part of yellow ferric oxide and 1 part of magnesium stearate were then mixed therewith. The mixed powder was made into a tablet using a rotary tableting machine at a rate of 120 mg per tablet. It was stored at 25°C and a relative humidity of 75% for 18 hours, and then stored at 30°C and a relative humidity of 40% for 4 hours to obtain an orally disintegrating tablet of the preparation of the present invention.

### Example 9

The same process as in Example 8 was carried out, except that the amount of methyl hesperidin was changed to 3 parts, to obtain an orally disintegrating tablet of the preparation of the present invention.

### Comparative Example 4

Ramosetron hydrochloride 0.0125 part
Crystalline cellulose (particles) 99 parts
Hydroxypropyl methylcellulose 1 part
1 part of hydroxypropyl methyl cellulose was dissolved in 10 parts of water under stirring using a magnetic stirrer, which were then stirred with 0.0125 part of ramosetron hydrochloride and 10 parts of methanol to prepare a pharmaceutical solution. 99 parts of crystalline cellulose (particles) was charged in a fluidized bed granulator (a product name: FLOW COATER, manufactured by Freund Corporation) equipped with a bug filter, and the pharmaceutical solution was side-sprayed at a spraying rate of 5 to 10 g/min to obtain a comparative particle preparation of the preparation of the present invention.

### Comparative Example 5

| | |
|---|---|
| Ramosetron hydrochloride | 0.01125 part |
| Crystalline cellulose (particles) | 89 parts |
| Hydroxypropyl methylcellulose | 2 parts |
| Mannitol | 9 parts |

2 parts of hydroxypropyl methyl cellulose was dissolved in 20 parts of water under stirring using a magnetic stirrer, which were then stirred with 9 parts of mannitol and 20 parts of methanol to prepare a coating solution. 90 parts of the particle preparation of Comparative Example 4 was charged in a fluidized bed granulator (a product name: FLOW COATER, manufactured by Freund Corporation) equipped with a bug filter, and the coating solution was side-sprayed at a spraying rate of 5 to 10 g/min to obtain a comparative particle preparation of the preparation the present invention.

### Comparative Example 6

| | |
|---|---|
| Ramosetron hydrochloride | 0.0124 part |
| Crystalline cellulose (particles) | 98 parts |
| Hydroxypropyl methylcellulose | 1.73 parts |
| Food Red No. 3 | 0.25 part |

0.74 part of hydroxypropyl methyl cellulose was dissolved in 48 parts of water under stirring using a magnetic stirrer, which were then stirred with 0.25 part of Food Red No. 3 to prepare a coating solution. 99 parts of the particle preparation of Comparative Example 4 was charged in a fluidized bed granulator (a product name: FLOW COATER, manufactured by Freund Corporation) equipped with a bug filter, and the coating solution was side-sprayed at a spraying rate of 5 to 10 g/min to obtain a comparative particle preparation of the preparation of the present invention.

### Comparative Example 7

| | |
|---|---|
| Ramosetron hydrochloride | 0.00125 part |
| Crystalline cellulose (particles) | 9.9 parts |
| Hydroxypropyl methylcellulose | 0.1 part |
| Mannitol | 81.8 parts |
| Maltose | 8.2 parts |

8.2 parts of maltose was dissolved in 32.8 parts of water under stirring using a magnetic stirrer to prepare a spraying liquid. 81.8 parts of mannitol was charged in a fluidized bed granulator (a product name: FLOW COATER, manufactured by Freund Corporation) equipped with a bug filter, and the spraying liquid was sprayed at a spraying rate of 10 g/min to conduct a fluid granulation. The granulated product and 10 parts of the particle preparation of Comparative Example 4 were mixed in a polyethylene bag, and the mixture was made into a tablet using autograph (AGS-20kNG, manufactured by Shimadzu Co.) at a rate of 200 mg per tablet to obtain a comparative tablet of the preparation of the present invention.

### Example 10

| | |
|---|---|
| Ramosetron hydrochloride | 0.01 part |
| Crystalline cellulose (particles) | 86 parts |
| Hydroxypropyl methylcellulose | 4.5 parts |
| αG Hesperidin | 9.4 parts |

3.7 parts of hydroxypropyl methyl cellulose was dissolved in 209 parts of water under stirring using a magnetic stirrer, which were then stirred with 9.4 parts of αG hesperidin to prepare a coating solution. 87 parts of the particle preparation of Comparative Example 4 was charged in a fluidized bed granulator (a product name: FLOW COATER, manufactured by Freund Corporation) equipped with a bug filter, and the coating solution was side-sprayed at a spraying rate of 5 to 10 g/min to obtain a particle preparation of the preparation of the present invention.

### Example 11

| | |
|---|---|
| Ramosetron hydrochloride | 0.0124 part |
| Crystalline cellulose (particles) | 98 parts |
| Hydroxypropyl methylcellulose | 1.73 parts |
| Food Red No. 102 | 0.25 part |

0.74 part of hydroxypropyl methyl cellulose was dissolved in 48 parts of water under stirring using a magnetic stirrer, which were then stirred with 0.25 part of Food Red No. 102 to prepare a coating solution. 99 parts of the particle preparation of Comparative Example 4 was charged in a fluidized bed granulator (a product name: FLOW COATER, manufactured by Freund Corporation) equipped with a bug filter, and the coating solution was side-sprayed at a spraying rate of 5 to 10 g/min to obtain a particle preparation of the preparation of the present invention.

### Example 12

| | |
|---|---|
| Ramosetron hydrochloride | 0.00124 part |
| Crystalline cellulose (particles) | 9.8 parts |
| Hydroxypropyl methylcellulose | 0.17 part |
| Food Red No. 102 | 0.025 part |
| Mannitol | 81.8 parts |
| Maltose | 8.2 parts |

8.2 parts of maltose was dissolved in 32.8 parts of water under stirring using a magnetic stirrer to prepare a spraying liquid. 81.8 parts of mannitol was charged in a fluidized bed granulator (a product name: FLOW COATER, manufactured by Freund Corporation) equipped with a bug filter, and the spraying liquid was sprayed at a spraying rate of 10 g/min to conduct a fluid granulation. The granulated product and 10 parts of the particle preparation of Example 11 were mixed in a polyethylene bag, and the mixture was made into a tablet using autograph (AGS-20kNG, manufactured by Shimadzu Co.) at a rate of 200 mg per tablet to obtain a tablet of the preparation of the present invention.

### Comparative Example 8

| | |
|---|---|
| Ramosetron hydrochloride | 0.0008 part |
| Mannitol | 89 parts |
| Maltose | 10 parts |
| Magnesium stearate | 1 part |

10 parts of maltose and 0.0008 part of ramosetron hydrochloride were dissolved in 67 parts of water under stirring using a magnetic stirrer to prepare a spraying liquid. Next, 90 parts of mannitol was put into a fluidized bed granulator (FLOW COATER, manufactured by Freund Corporation), and the spraying liquid was sprayed thereonto at a spraying rate of 10 g/min to conduct a fluid granulation. After granulation, 1 part of magnesium stearate was mixed therewith. The mixed powder was made into a tablet using a rotary tableting machine at a rate of 120 mg per tablet. It was stored at 25°C and a relative humidity of 75% for 18 hours, and then stored at 30°C and a relative humidity of 40% for 4 hours to obtain a comparative orally disintegrating tablet of the preparation of the present invention.

### Example 13

| | |
|---|---|
| Ramosetron hydrochloride | 0.002 part |
| Mannitol | 91 parts |
| Maltose | 6.9 parts |
| αG Hesperidin | 1 part |
| Magnesium stearate | 1 part |

1.7 parts of maltose and 0.002 part of ramosetron hydrochloride were dissolved in 6.8 parts of water using a magnetic stirrer under stirring to prepare a spraying liquid. Next, 91 parts of mannitol was put into a fluidized bed granulator (GPCG-1, manufactured by Powrex Corporation) equipped with a bug filter, and the spraying liquid was sprayed thereonto at an inlet temperature of 63°C, a spraying rate of 15 g/min, and a spraying/drying cycle of 12 seconds/24 seconds to conduct a fluid granulation. Further, a spraying liquid obtained by dissolving 5.1 parts of maltose and 1 part of αG hesperidin in 20.4 parts of water was sprayed to the granulated product under the same condition to conduct a fluid granulation. After granulation, the granulated product was dried for 1 minute, and 1 part of magnesium stearate was then mixed therewith. The mixed powder was made into a tablet using a rotary tableting machine at a rate of 120 mg per tablet. It was stored at 25°C and a relative humidity of 75% for 18 hours, and then stored at 30°C and a relative humidity of 40% for 4 hours to obtain an orally disintegrating tablet of the preparation of the present invention.

### Example 14

| | |
|---|---|
| Ramosetron hydrochloride | 0.002 part |
| Mannitol | 91 parts |
| Maltose | 6.9 parts |
| Methyl hesperidin | 1 part |
| Magnesium stearate | 1 part |

1.7 parts of maltose and 0.002 part of ramosetron hydrochloride were dissolved in 6.8 parts of water using a magnetic stirrer under stirring to prepare a spraying liquid. Next, 91 parts of mannitol was put into a fluidized bed granulator (GPCG-1, manufactured by Powrex Corporation) equipped with a bug filter, and the spraying liquid was sprayed thereonto at an inlet temperature of 63°C, a spraying rate of 15 g/min, and a spraying/drying cycle of 12 seconds/24 seconds to conduct a fluid granulation. Further, a spraying liquid obtained by dissolving 5.1 parts of maltose and 1 part of methyl hesperidin in 20.4 parts of water was sprayed to the granulated product under the same condition to conduct a fluid granulation. After granulation, the granulated product was dried for 1 minute, and 1 part of magnesium stearate was then mixed therewith. The mixed powder was made into a tablet using a rotary tableting machine at a rate of 120 mg per tablet. It was stored at 25°C and a relative humidity of 75% for 18 hours, and then stored at 30°C and a relative humidity of 40% for 4 hours to obtain an orally disintegrating tablet of the preparation of the present invention.

### <Evaluation on stability>

The stabilizing effect of the preparation of the present invention was evaluated by storing the preparation of the present invention under various storage conditions (under irradiation with a white fluorescent lamp of 1000 Lux, under sealing of an HDPE plastic bottle at 40°C and 75% RH), and after passing for a certain period of time, calculating a assay value of the stored product under various conditions against the assay value of the stored product before storage or after light shielding at 5°C of the preparation of the present invention. The quantitative determination was carried out by liquid chromatography.

### <Results and Consideration>

With respect to a 2.5 µg tablet of ramosetron hydrochloride added with a compound having a different light absorbance behavior, or a 2.5 µg tablet of ramosetron hydrochloride not added with the compound, the stability of ramosetron hydrochloride in each of the preparations under irradiation with light was evaluated. The results are shown in Table 1.

**[Table 1]**

| Storage condition and Storage period | Assay value (%) | | | | | | |
|---|---|---|---|---|---|---|---|
| | Comparative Example 1 | Comparative Example 2 | Comparative Example 3 | Example 1 | Example 2 | Example 3 | Example 4 |
| 1000 Lux, for 2 weeks | 21 | 54 | 68 | 97 | 98 | 97 | 92 |

In the 2.5 µg tablet of ramosetron hydrochloride of Comparative Example 1, a lowering of the assay value was found.
In the 2.5 µg tablet of ramosetron hydrochloride as prepared by adding Food Red No. 3 of Comparative Example 2 in the granulation process, and the 2.5 µg tablet of ramosetron hydrochloride as prepared by adding rutin of Comparative Example 3 in the blending process, the degree of a lowering of the assay value is not significantly improved, and the drug stabilization effect due to the light absorbance base is not sufficient.
In contrast, the 2.5 µg tablet of ramosetron hydrochloride as prepared by adding αG hesperidin of Example 1 in the granulation process, the 2.5 µg tablet of ramosetron hydrochloride as prepared by adding Food Red No. 102 of Example 2 in the granulation process, the 2.5 µg tablet of ramosetron hydrochloride as prepared by adding methyl hesperidin of Example 3 in the granulation process, and the 2.5 µg tablet of ramosetron hydrochloride as prepared by adding sodium azulene sulfonate of Example 4 in the granulation process, a change of the assay value was not substantially found as compared with the product that was stored under light shielding at 5°C.
From these results, it has become clear that by adding αG hesperidin, methyl hesperidin, Food Red No. 102, and sodium azulene sulfonate to ramosetron hydrochloride, a remarkable stabilizing effect of ramosetron hydrochloride against the irradiation with light is found.

Furthermore, the relationship between the light absorbance behavior of the compound used herein, and the stabilizing effect for ramosetron hydrochloride were specifically analyzed.
As a result, it was found that the light absorbance spectrum of ramosetron had three peaks showing at maximum absorbance wavelengths at 210 nm, 249 nm, and 311 nm (see Fig. 1). In addition, from the results of Fig. 1, the area under the spectrum curve was derived from the compound used herein, and analyzed (see Fig. 2). Thus, surprisingly, it became apparent that the area under the spectrum curve at the wavelength range of 220 nm to 240 nm, and/or the area under the spectrum curve at the wavelength range of 280 nm to 300 nm, of the compound to be added, is/are important for stabilization of ramosetron hydrochloride, and thus, it was found that addition of the compound characterized by 4.5 or more of the area under the spectrum curve at the wavelength range of 220 nm to 240 nm, and/or 2.5 or more of the area under the spectrum curve at the wavelength range of 280 nm to 300 nm in a 0.001 w/v% aqueous solution thereof is extremely effective (see Figs. 3 and 4). Further, in Comparative Example 1, the area under the spectrum curve at the wavelength range of 220 nm to 240 nm and a range of 280 nm to 300 nm in a 0.001 w/v% aqueous solution thereof of the compound to be added in each Comparative Example and Example was 0 since it does not contain a compound absorbing a light having a specific wavelength, and the areas of Food Red No. 3 (Comparative Example 2) were 4.2 (220 nm to 240 nm) and 1.8 (280 nm to 300 nm), respectively, the areas of rutin (Comparative Example 3) were 4.4 (220 nm to 240 nm) and 2.4 (280 nm to 300 nm), respectively, the areas of αG hesperidin (Example 1) were 6.0 (220 nm to 240 nm) and 5.0 (280 nm to 300 nm), respectively, the areas of Food Red No. 102 (Example 2) were 10.0 (220 nm to 240 nm) and 3.1 (280 nm to 300 nm), respectively, the areas of methyl hesperidin (Example 3) were 5.4 (220 nm to 300 nm) and 3.7 (280 nm to 300 nm), respectively, and the areas of sodium azulene sulfonate (Example 4) were 8.4 (220 nm to 240 nm) and 19.9 (280 nm to 300 nm).

Next, with respect to a 2.5 µg tablet of ramosetron hydrochloride prepared by changing the blending ratio of the αG hesperidin as a compound that has been confirmed to have a stabilizing effect against light of ramosetron, and methyl hesperidin, a 2.5 µg tablet of ramosetron prepared by adding a trace amount of yellow ferric oxide in addition to a compound exhibiting stabilization, or a 2.5 µg tablet of ramosetron not containing a compound absorbing light having a specific wavelength, the stability of ramosetron hydrochloride in each of the preparations under irradiation with light was evaluated. The results are shown in Table 2.

**[Table 2]**

| Storage condition and Storage period | Assay value (%) | | | | | |
|---|---|---|---|---|---|---|
| | Comparative Example 1 | Example 5 | Example 6 | Example 7 | Example 8 | Example 9 |
| 1000 Lux, for 2 weeks | 21 | 80 | 98 | 90 | 92 | 87 |

In the 2.5 µg tablet of ramosetron hydrochloride of Comparative Example 1, a lowering of the assay value was found.
In contrast, in the 2.5 µg tablet of ramosetron hydrochloride as prepared by adding αG hesperidin of Examples 5 and 6 in the granulation process, a little lowering of the assay value at an addition amount of αG hesperidin of 0.1%, was found as compared with the product that was stored under light shielding at 5°C, but substantially no change in the assay values at an addition amount of αG hesperidin of 3% was found as compared with the product that was stored under light shielding at 5°C. Similarly, in the 2.5 µg tablet of ramosetron hydrochloride as prepared by adding 3% of methyl hesperidin of Example 7, substantially no change in the assay value was found as compared with the product that was stored under light shielding at 5°C.
From these results, it has become clear that without a significant influence of the addition amount of αG hesperidin or methyl hesperidin that is a compound absorbing light having a specific wavelength, a remarkable stabilizing effect of ramosetron hydrochloride against the irradiation with light is found.
Further, for the 2.5 µg tablet of ramosetron hydrochloride as prepared by adding a trace amount of yellow ferric oxide as a coloring agent to methyl hesperidin that is a compound exhibiting stabilization against light of Examples 8 and 9, though the addition amount of yellow ferric oxide is as much low as 0.1%, a stabilizing effect of ramosetron hydrochloride against the irradiation with light was found. Accordingly, it is thought that a compound absorbing light having a specific wavelength that improves the stability of ramosetron against light can be used in combination with a yellow ferric oxide as a coloring material, reduction of the addition amount thereof can be realized, and reduction of the problems of adhesion, and the like becomes possible.

With respect to the uncoated ramosetron hydrochloride granulates and the ramosetron hydrochloride granulates having different light absorbance behaviors coated with various compounds, the stability of ramosetron hydrochloride in each of the preparations under irradiation with light was evaluated. The results are shown in Table 3.

**[Table 3]**

| Storage condition and Storage period | Assay value (%) | | | | |
|---|---|---|---|---|---|
| | Comparative Example 4 | Comparative Example 5 | Comparative Example 6 | Example 10 | Example 11 |
| 1000 Lux, for 2 weeks | 20 | 24 | 6 | 92 | 92 |

In the uncoated ramosetron hydrochloride granulates of Comparative Example 4, a compound absorbing light having a specific wavelength was not coated, and thus a lowering of the assay value was found. Further, the areas under the spectrum curve at the wavelength range of 220 nm to 240 nm and in a range of 280 nm to 300 nm of the compound added in each of Comparative Example in a 0.001 w/v% aqueous solution thereof are, for mannitol (Comparative Example 5), 0.1 (220 nm to 240 nm) and 0.1 (280 nm to 300 nm), for Food Red No. 3 (Comparative Example 6), 4.2 (220 nm to 240 nm) and 1.8 (280 nm to 300 nm), and are not sufficient absorbance areas exhibiting the stabilizing effect of ramosetron against light, and in this regard, although coating was carried out for the preparations of Comparative Examples 5 and 6, a remarkable improvement in the assay values could not be seen.
For these, when coating was carried out using αG hesperidin of Example 10 and Food Red No. 102 of Example 11, there was no substantial change in the assay values from that of ramosetron hydrochloride before storage.
From these results, it has become clear that by coating the granules containing ramosetron hydrochloride with αG hesperidin and Food Red No. 102, a remarkable stabilizing effect of ramosetron hydrochloride against the irradiation with light is found.

Next, with respect to the uncoated ramosetron hydrochloride granulates and the 2.5 µg tablet of ramosetron hydrochloride comprising ramosetron hydrochloride granules coated with Food Red No. 102, the stability of ramosetron hydrochloride in each of the preparations under irradiation with light was evaluated. The results are shown in Table 4.

**[Table 4]**

| Storage condition and storage period | Assay value (%) | |
|---|---|---|
| | Comparative Example 7 | Example 12 |
| 1000 Lux, for 2 weeks | 30 | 95 |

In the 2.5 µg tablet of ramosetron hydrochloride containing the uncoated ramosetron hydrochloride granulates of Comparative Example 7, a lowering of the assay value was found.
In contrast, in the 2.5 µg tablet of ramosetron hydrochloride containing the ramosetron hydrochloride granulates coated with Food Red No. 102 of Example 12, substantially no change in the assay values was found as compared with the initial value.
From these results, it has become clear that when a tablet was prepared using the ramosetron hydrochloride granulates coated with Food Red No. 102, a remarkable stabilizing effect of ramosetron hydrochloride against the irradiation with light is found.
As apparent from Tables 1, 2, 3, and 4, and the like, irrespective of the content of ramosetron hydrochloride contained in the preparations, and further, irrespective of the types of the preparation and the addition process of the light-stable compound, it was found that the compound characterized by 4.5 or more of the area under the spectrum curve at the wavelength range of 220 nm to 240 nm, and/or 2.5 or more of the area under the spectrum curve at the wavelength range of 280 nm to 300 nm in a 0.001 w/v% aqueous solution thereof contributes to stabilization of the ramosetron hydrochloride preparation against irradiation with light.

Next, investigation was made on whether αG hesperidin and methyl hesperidin having a stabilizing effect against irradiation with light contributes to stabilization against temperature/humidity. While excluding citric acid known to have a stabilizing effect of ramosetron hydrochloride against a temperature and a humidity from the formulation, with respect to a 2.5 µg tablet of ramosetron hydrochloride containing αG hesperidin or methyl hesperidin, and a 2.5 µg tablet of ramosetron hydrochloride not added with these compounds, the stability of ramosetron hydrochloride in each of the preparations under a temperature/humidity condition was evaluated. The results are shown in Table 5.

**[Table 5]**

| Storage condition and Storage period | Assay value (%) | | |
|---|---|---|---|
| | Comparative Example 8 | Example 13 | Example 14 |
| Under shielding of bottle at 40°C and 75% RH for 2 weeks | No data | 95 | 68 |
| Under shielding of bottle at 40°C and 75% RH for 1 month | 33 | 88 | 66 |

In the 2.5 µg tablet of ramosetron hydrochloride of Comparative Example 8, a lowering of the assay value was found.
In contrast, in the 2.5 µg tablet of ramosetron obtained by blending αG hesperidin of Example 13, substantially no change in the assay values was found as compared with the product that was stored under light shielding at 5°C. On the other hand, in the 2.5 µg tablet of ramosetron obtained by blending methyl hesperidin of Example 14, a little lowering in the assay values was found as compared with the product that was stored under light shielding at 5°C, but improvement in the assay values was found as compared with the 2.5 µg tablet of ramosetron not added with the compound to be stabilized of Comparative Example 8. From these results, it has become clear that a stabilizing effect of the ramosetron tablet obtained by blending αG hesperidin and methyl hesperidin against the irradiation with light, as well as against the temperature/humidity is found.

### INDUSTRIAL AVAILABILITY

The solid pharmaceutical composition of the present invention can provide a stable preparation by blending a compound absorbing a light having a specific wavelength with ramosetron which is unstable, usually under irradiation with light, or a pharmaceutically acceptable salt thereof. Particularly, it is useful as a technique that is adaptable to a preparation containing ramosetron or a pharmaceutically acceptable salt thereof at a low content or an orally disintegrating tablet containing ramosetron or a pharmaceutically acceptable salt thereof.

## Claims

1. A solid pharmaceutical composition of ramosetron or a pharmaceutically acceptable salt thereof, which contains one or two or more kinds of the compounds selected from the group consisting of the compounds having 4.5 or more of the area under the spectrum curve at the wavelength range of 220 nm to 240 nm and/or 2.5 or more of the area under the spectrum curve at the wavelength range of 280 nm to 300 nm in a 0.001 w/v% aqueous solution thereof.

2. The pharmaceutical composition according to claim 1, wherein the compounds having 4.5 or more of the area under the spectrum curve at the wavelength range of 220 nm to 240 nm, and/or 2.5 or more of the area under the spectrum curve at the wavelength range of 280 nm to 300 nm in a 0.001 w/v% aqueous solution thereof is αG hesperidin, methyl hesperidin, Food Red No. 102, or sodium azulene sulfonate.

3. The pharmaceutical composition according to claim 1 or 2, wherein the blending amount of one, or two or more kinds of the compounds selected from the group consisting of the compounds having 4.5 or more of the area under the spectrum curve at the wavelength range of 220 nm to 240 nm and/or 2.5 or more of the area under the spectrum curve at the wavelength range of 280 nm to 300 nm in a 0.001 w/v% aqueous solution thereof is from 0.001 to 90% by weight in the formulation.

4. The pharmaceutical composition according to claim 3, wherein the blending amount of ramosetron or a pharmaceutically acceptable salt thereof is from 0.0001 to 0.5% by weight in the formulation.

5. A particulate pharmaceutical composition, wherein ramosetron or a pharmaceutically acceptable salt thereof is coated with one, or two or more kinds of the compounds selected from the group consisting of the compounds having 4.5 or more of the area under the spectrum curve at the wavelength range of 220 nm to 240 nm and/or 2.5 or more of the area under the spectrum curve at the wavelength range of 280 nm to 300 nm.

6. An orally disintegrating tablet, which contains the pharmaceutical composition as described in any one of claims 1 to 5.

7. The pharmaceutical composition according to any one of claims 1 to 6, which contains one, or two or more selected from the group consisting of yellow ferric oxide, red ferric oxide, and titanium oxide in an amount of 0.0001 to 0.5% by weight in the formulation.

8. A method for stabilizing a solid pharmaceutical composition of ramosetron or a pharmaceutically acceptable salt thereof, which comprises blending one or two or more kinds of the compounds selected from the group consisting of the compounds having 4.5 or more of the area under the spectrum curve at the wavelength range of 220 nm to 240 nm and/or 2.5 or more of the area under the spectrum curve at the wavelength range of 280 nm to 300 nm in a 0.001 w/v% aqueous solution thereof.
